# FASCICULE DE BREVET EUROPEEN

(11) **EP 3 075 413 B1**
(45) Date de publication et mention de la délivrance du brevet: **06.02.2019**
(21) Numéro de dépôt: 16162556.1
(22) Date de dépôt: 29.03.2016
(51) Int. Cl.: A61N 1/368, A61N 1/05, A61B 5/042

(54) **DISPOSITIF MÉDICAL IMPLANTABLE ACTIF À STIMULATION BIVENTRICULAIRE, COMPRENANT DES MOYENS DE MESURE DU TEMPS DE CONDUCTION INTERVENTRICULAIRE POUR LE DIAGNOSTIC DE LA DÉCOMPENSATION CARDIAQUE**
IMPLANTIERBARES MEDIZINISCHES GERÄT FÜR DIE BIVENTRIKULÄRE STIMULATION, UMFASSEND MITTEL ZUR MESSUNG INTRAVENTRIKULÄRER LEITUNGSZEIT FÜR DIE DIAGNOSE VON HERZDEKOMPENSATION
IMPLANTABLE MEDICAL DEVICE FOR BIVENTRICULAR STIMULATION, INCLUDING MEANS FOR MEASURING INTRAVENTRICULAR CONDUCTION TIME FOR DIAGNOSIS OF HEART DECOMPENSATION

(30) Priorité: 02.04.2015 FR 1552843
(43) Date de publication de la demande: 05.10.2016
(73) Titulaire: Sorin CRM SAS, 92140 Clamart Cedex (FR)
(72) Inventeur: EUZEN, Marie-Anne, 91570 BIEVRES (FR); MILPIED, Paola, 75014 PARIS (FR)
(74) Mandataire: Grünecker Patent- und Rechtsanwälte PartG mbB

(56) Documents cités:
- EP-A1- 2 105 843
- EP-A1- 2 324 885
- EP-A1- 2 357 020
- EP-A1- 2 368 493
- EP-A1- 2 737 925
- EP-A1- 2 742 971
- EP-A1- 2 742 973
- EP-A1- 2 839 859
- US-A1- 2006 253 164
- US-A1- 2008 294 217

## Description

L'invention concerne les "dispositifs médicaux implantables actifs" tels que définis par la directive 90/385/CEE du 20 juin 1990 du Conseil des communautés européennes.

Elle concerne plus précisément les implants permettant de surveiller en continu le rythme cardiaque et délivrer si nécessaire au coeur des impulsions électriques destinées à stimuler de façon conjointe et permanente les ventricules gauche et droit afin de resynchroniser ces derniers, technique dite "CRT" (*Cardiac Resynchronization Therapy*) ou "BVP" (*BiVentricular Pacing*).

Un stimulateur CRT est par exemple divulgué dans le EP 1 108 446 A1 (ELA Medical), qui décrit un dispositif permettant d'appliquer entre les instants respectifs de stimulation des ventricules gauche et droit un délai dit "délai interventriculaire" (DW ou WD) variable, ajusté de manière à resynchroniser la contraction des ventricules avec optimisation fine de l'état hémodynamique du patient. Le DVV pourra être nul, positif (le ventricule gauche étant stimulé après le ventricule droit), ou négatif (le ventricule droit étant stimulé après le ventricule gauche).

Les dispositifs CRT incluent en outre un mode de fonctionnement "double chambre" classique dans lequel le dispositif surveille l'activité ventriculaire après un événement auriculaire spontané (détection d'une onde P de dépolarisation auriculaire) ou stimulé (application d'une impulsion A de stimulation auriculaire). En même temps, le dispositif commence à compter un délai dit "délai atrioventriculaire" ou "délai auriculo-ventriculaire" (DAV ou AVD) tel que si aucune activité spontanée ventriculaire (onde R) n'a été détectée à l'issue de ce délai, alors le dispositif déclenche une stimulation de ce ventricule (application d'une impulsion V).

Le but général de la présente invention est, dans un tel contexte, de mesurer le temps de conduction interventriculaire, c'est-à-dire l'intervalle de temps séparant une stimulation appliquée à l'un des ventricules de la contraction spontanée induite dans l'autre ventricule par cette stimulation (on notera que dans cette configuration particulière de mesure, les deux ventricules ne sont plus stimulés conjointement).

Les modifications de ce paramètre sur le long terme sont en effet un bon indicateur de l'évolution de l'état cardiaque du patient, notamment du phénomène dénommé "remodelage cardiaque", qui peut être défini comme l'ensemble des modifications du coeur engendrées en réponse à une pathologie, et qui est généralement associé à un plus mauvais pronostic.

Les modifications cliniques pouvant être asymptomatiques, il est courant que le patient adapte inconsciemment son activité à son état clinique : les premières crises d'insuffisance cardiaque apparaissant à l'effort, ceci conduit le patient à réduire son activité pour éviter la survenue de telles crises. Ensuite, les symptômes n'apparaissent plus, le patient ayant modifié son comportement pour les éviter, mais la pathologie continue à progresser.

Le remodelage se manifeste à la longue par une augmentation du volume du ventricule gauche, avec dégradation de la fraction d'éjection et du régime des pressions intraventriculaires du fait de la baisse de contractilité et/ou de pressions trop élevées en aval, et *in fine* une diminution du débit cardiaque entrainant de graves conséquences sur l'organisme par progression de l'insuffisance cardiaque. Et ce n'est que lorsque l'insuffisance cardiaque gênera le patient même au repos qu'il ira consulter ou, dans les cas extrêmes, devra être hospitalisé en urgence.

En résumé, du fait de cette auto-adaptation, l'absence de symptômes ressentis introduit un retard important entre le début des modifications cliniques et le diagnostic de ces modifications, souvent trop tardif.

En stimulant de façon contrôlée les ventricules en au moins deux points, la thérapie CRT permet d'optimiser le cycle contraction/relaxation, avec un bénéfice direct facilitant le travail du coeur, ce qui peut permettre de stabiliser le phénomène de remodelage, et même le contrer ("remodelage inverse") avec pour le patient un meilleur pronostic.

Le but principal de l'invention est de proposer un moyen de diagnostic, incorporé à un stimulateur CRT, qui permette d'assurer un suivi régulier (par exemple quotidien) de l'état du patient, en particulier l'évolution d'un remodelage cardiaque ou d'un remodelage inverse, et ceci de façon suffisamment précoce pour pouvoir prendre au plus vite les mesures appropriées - par exemple une modification du paramétrage de la thérapie CRT, ou la substitution à cette thérapie CRT d'une autre thérapie si elle n'est pas efficace -, évitant ainsi le déclenchement imprévu d'une crise à brève ou moyenne échéance.

La technique de référence pour l'évaluation de l'état du patient, et donc pour l'évaluation de la thérapie CRT et l'ajustement des paramètres de stimulation CRT, est l'évaluation par échocardiographie avec mesure du volume ventriculaire et estimation des délais caractéristiques de la systole, en particulier du temps d'ouverture de la valve aortique. Cette procédure doit toutefois être mise en oeuvre en milieu hospitalier et par un personnel qualifié, elle est longue et couteuse et ne peut pas être appliquée aussi souvent que cela serait utile ou nécessaire sans interférer avec la vie quotidienne du patient.

La mesure à intervalles réguliers, par exemple quotidiennement, du temps de conduction interventriculaire et le suivi sur le long terme de cette donnée permettent de diagnostiquer un remodelage ventriculaire inverse (favorable) indiquant que la thérapie CRT est efficace, ou inversement un remodelage ventriculaire (délétère) indiquant une dégradation de l'état du patient.

Des techniques d'analyse automatique à partir du dispositif implanté ont également été proposées, par exemple par le US 2011/0152660 A1, où la dégradation et l'amélioration du patient sont évaluées à partir de l'évolution du délai interventriculaire, considéré seul ou en combinaison avec l'évaluation de l'activité du patient et/ou de la présence d'oedème pulmonaire. Le temps de conduction interventriculaire est évalué au moyen d'électrodes ventriculaires endocardiaques ou épicardiques, à gauche et à droite. Cette méthode est cependant très sensible à la position des sondes, une variation de par exemple 20 % du temps de conduction interventriculaire étant assimilée à la détection du déplacement d'une sonde. Cette technique est également sensible à la manière dont le coeur se remodèle, de sorte que dans certains cas la mesure du temps de conduction interventriculaire peut ne pas apporter l'information recherchée de remodelage, notamment si aucun changement significatif du délai de conduction n'est observé selon l'axe de mesure (entre l'électrode unique située à droite et l'électrode unique située à gauche) - alors que selon un autre axe une variation beaucoup plus importante aurait pu être observée. Le US 2007/0239037 A1 décrit une autre technique basée sur la mesure du temps de conduction interventriculaire (déterminé par échographie) avant l'implantation du dispositif, et qui a pour objet de prédire la réponse d'un patient à une thérapie CRT : plus grand est le temps de conduction interventriculaire initial, plus la probabilité d'avoir un remodelage important est élevée. Il s'agit toutefois d'un outil de prédiction nécessitant une échographie à un instant initial, et non d'un outil de diagnostic permettant d'évaluer sur le long terme l'évolution d'un état du patient, postérieurement à l'implantation du dispositif CRT.

Le US 2007/0043394 A1 décrit une technique de diagnostic de la décompensation cardiaque par détection d'une variation de l'impédance intracardiaque, à fréquence cardiaque constante. L'évolution de ce paramètre sur le long terme permet de déduire si le coeur se remodèle ou non. La mesure de l'impédance intracardiaque n'est toutefois pas un paramètre stable sur le long terme (plusieurs semaines, voire plusieurs mois), ce qui en rend difficile l'exploitation à des fins diagnostiques avec un degré de fiabilité suffisant.

Le but de l'invention est de proposer un dispositif de type implant CRT pourvu de moyens de diagnostic du remodelage cardiaque permettant de pallier les inconvénients précités, et en particulier :
- de tenir compte d'au moins deux axes de mesure afin d'obtenir une mesure plus robuste du délai interventriculaire (mesure qui sera désignée "mesure multidimensionnelle du temps de conduction interventriculaire") ;
- d'éviter des examens longs et couteux par échographie cardiaque, qui ne fournissent qu'un diagnostic tardif, souvent plusieurs mois après l'implantation ;
- d'évaluer de façon précoce et robuste le remodelage du patient afin d'adapter en conséquence la thérapie de resynchronisation (réajustement éventuel des DAV et/ou DW) ;
- de mettre en place des alertes en cas de risques de décompensation aggravée ou d'ischémie, pour prévenir les hospitalisations et modifier en temps utiles les thérapies et traitements ; et
- de façon générale, de s'assurer de la bonne délivrance et de l'efficacité de la thérapie CRT.

Plus précisément, l'invention propose un dispositif de resynchronisation cardiaque par stimulation biventriculaire comprenant, de manière en elle-même connue :
- des moyens de détection d'événements auriculaires et ventriculaires ;
- des moyens de stimulation des ventricules droit et gauche, avec application i) d'un délai atrioventriculaire, DAV, entre un événement auriculaire spontané ou stimulé et une stimulation du ventricule droit, et ii) d'un délai interventriculaire, DW, entre les instants respectifs de stimulation des ventricules droit et gauche ;
- des moyens de mesure multidimensionnelle du temps de conduction interventriculaire, compté entre une stimulation du ventricule droit ou gauche et un événement spontané détecté dans le ventricule gauche, ou *vice versa,* ou entre une stimulation du ventricule droit et un évènement spontané détecté entre ventricules droit et gauche, au cours d'un même cycle cardiaque ; et
- des moyens de suivi de l'évolution d'un état du patient, aptes à mémoriser un historique des temps de conduction mesurés à des dates successives et à en dériver un indice représentatif.

De façon caractéristique de l'invention, les moyens de mesure multidimensionnelle du temps de conduction interventriculaire comprennent :
- des moyens aptes à commander pendant au moins un cycle cardiaque les moyens de stimulation et les moyens de détection de manière à :
   - produire une stimulation de l'un des ventricules, et
   - recueillir dans l'autre ventricule, concurremment sur des voies respectives distinctes, au moins deux signaux EGM d'électrogramme endocavitaire et en dériver au moins deux composantes temporelles distinctes respectives ;
- des moyens aptes à combiner les au moins deux composantes temporelles en au moins une caractéristique 2D paramétrique représentative du cycle cardiaque, à partir des variations de l'une des composantes temporelles en fonction de l'autre ;
- des moyens d'analyse, aptes à dériver de la caractéristique 2D, ou d'une moyenne de caractéristiques 2D recueillies sur des cycles cardiaques successifs, un vecteur de paramètres descripteurs intrinsèques courants de la caractéristique 2D ; et
- des moyens comparateurs, aptes à comparer ledit vecteur de paramètres descripteurs courants à un vecteur homologue de paramètres descripteurs de référence, pour en dériver un indice représentatif de l'évolution d'un état du patient.

Selon diverses caractéristiques subsidiaires avantageuses :
- les paramètres descripteurs intrinsèques sont des paramètres fonction du vecteur vitesse de la caractéristique 2D considéré en une pluralité de points respectifs de cette caractéristique, notamment les valeurs respectives de la norme du vecteur vitesse ;
- les moyens comparateurs comprennent des moyens aptes à opérer une corrélation croisée entre i) le vecteur de paramètres descripteurs courants et ii) le vecteur de paramètres descripteurs de référence ;
- l'indice représentatif de l'évolution d'un état du patient est la valeur du retard qui maximise la fonction d'intercorrélation entre le vecteur de paramètres descripteurs courants et le vecteur de paramètres descripteurs de référence, ce retard pouvant notamment être comparé à un seuil prédéterminé pour déclencher une alerte en cas de franchissement de ce seuil ;
- le dispositif comprend en outre des moyens aptes à raccourcir temporairement le délai atrioventriculaire du dispositif pendant l'activation des moyens de mesure multidimensionnelle du temps de conduction interventriculaire ;
- le dispositif comprend en outre des moyens aptes à forcer temporairement l'activation de moyens de stimulation auriculaires pendant l'activation des moyens de mesure multidimensionnelle du temps de conduction interventriculaire ;
- les au moins deux signaux EGM recueillis concurremment sur des voies respectives distinctes comprennent :
   - un signal EGM *far-field* unipolaire (*Vuni*) recueilli entre i) une électrode proximale ou distale ou le cas échéant une électrode intermédiaire ou un bobinage de défibrillation d'une sonde ventriculaire située dans le ventricule non stimulé et ii) un boitier métallique de générateur du dispositif,
      ou bien entre i) une première électrode proximale, ou électrode distale ou bobinage de défibrillation et ii) et une seconde électrode proximale, ou une électrode distale ou le cas échéant une électrode intermédiaire ou un bobinage de défibrillation, respectivement de deux sondes ventriculaires distinctes situées toutes deux dans le ventricule non stimulé,
      ou bien entre i) une première électrode proximale, ou une électrode distale ou un bobinage de défibrillation et ii) et une seconde électrode proximale, ou une électrode distale ou une électrode intermédiaire, respectivement d'une sonde ventriculaire droite et d'une sonde ventriculaire gauche, et
   - un signal EGM *near-field* bipolaire recueilli entre i) une électrode distale et ii) une électrode proximale d'une sonde ventriculaire située dans le ventricule non stimulé,
      ou bien entre i) un bobinage de défibrillation et ii) une électrode distale ou proximale de ladite sonde ventriculaire,
      ou bien entre i) une électrode distale et ii) une électrode intermédiaire d'une sonde ventriculaire gauche,
      ou bien entre i) une électrode proximale et ii) une électrode intermédiaire de ladite sonde ventriculaire gauche,
      ou bien entre deux électrodes intermédiaires de ladite sonde ventriculaire gauche.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 est une vue générale montrant un dispositif CRT avec son générateur et des sondes cardiaques droite et gauche implantées dans le coeur.
La Figure 2 est un exemple de signaux EGM obtenus sur des voies respectivement ventriculaire bipolaire et ventriculaire unipolaire de l'une des sondes de la Figure 1.
La Figure 3 est un exemple montrant l'évolution, sur le long terme, des signaux EGM bipolaire et unipolaire obtenus en stimulant le ventricule gauche et en recueillant le signal correspondant dans le ventricule droit.
La Figure 4 est un autre exemple d'EGM, du même type que celui de la
Figure 3.

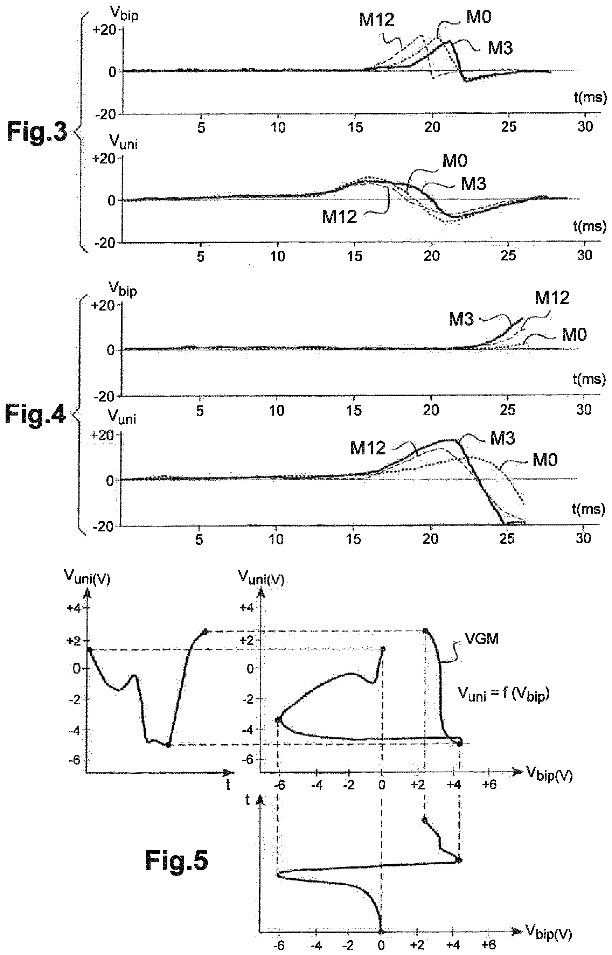

La Figure 5 illustre la manière de combiner entre eux les signaux bipolaire et unipolaire recueillis dans une même cavité ventriculaire pour construire une caractéristique bidimensionnelle de type vectogramme, indépendante du temps.

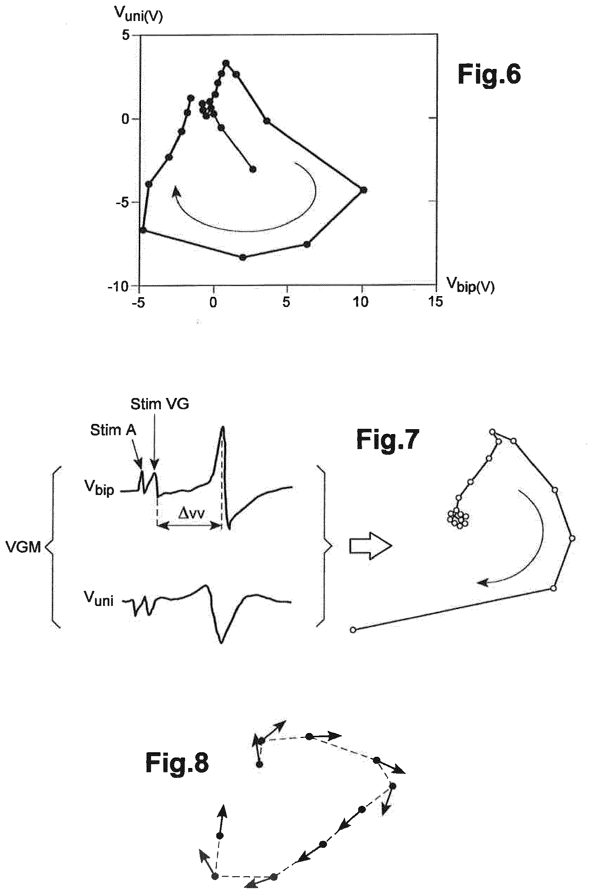

La Figure 6 est un exemple de vectogramme obtenu pour un cycle cardiaque échantillonné à 128 Hz.
La Figure 7 illustre des exemples de signaux bipolaire et unipolaire recueillis dans le ventricule droit suite à une stimulation appliquée au ventricule gauche, ainsi que le vectogramme correspondant construit à partir de ces signaux.
La Figure 8 est une représentation des vecteurs vitesse dans un exemple de vectogramme échantillonné.

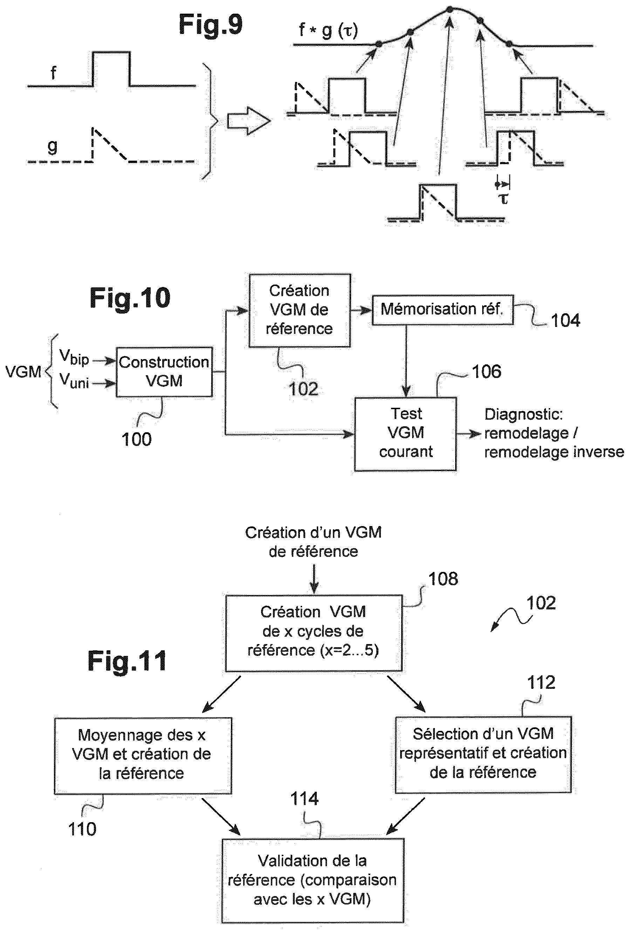

La Figure 9 illustre le principe d'un traitement de corrélation croisée, appliqué entre les paramètres descripteurs courants et des paramètres descripteurs de référence des vectogrammes analysés.
La Figure 10 est un schéma de principe illustrant la mise en oeuvre de l'invention.
La Figure 11 est un diagramme présentant la séquence d'étapes exécutées lors de l'élaboration préalable des références.

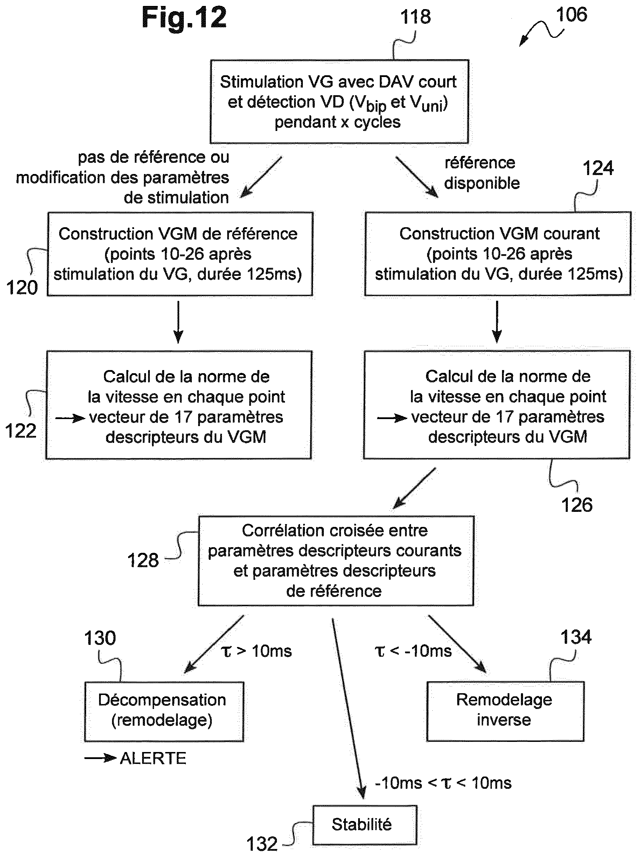

La Figure 12 est un diagramme présentant la séquence d'étapes exécutées lors du suivi du patient, pour déterminer l'évolution de son état, notamment d'un remodelage ou remodelage inverse.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu comprenant des moyens d'acquisition d'un signal fourni par des sondes endocavitaires et/ou par un ou plusieurs capteurs implantés.

L'invention peut notamment être appliquée aux dispositifs implantables tels que ceux des familles *Reply, Paradym, Intensia, Paradym RF* et *Platinium,* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé de l'invention est mis en oeuvre par des moyens principalement logiciels, grâce à des algorithmes appropriés exécutés automatiquement et de façon récurrente par un microcontrôleur ou un processeur numérique de signal. Pour la clarté de l'exposé, les divers traitements appliqués sont décomposés et schématisés par un certain nombre de blocs fonctionnels distincts, mais cette représentation n'a toutefois qu'un caractère illustratif, ces circuits comprenant des éléments communs et correspondant en pratique à une pluralité de fonctions globalement exécutées par un même logiciel.

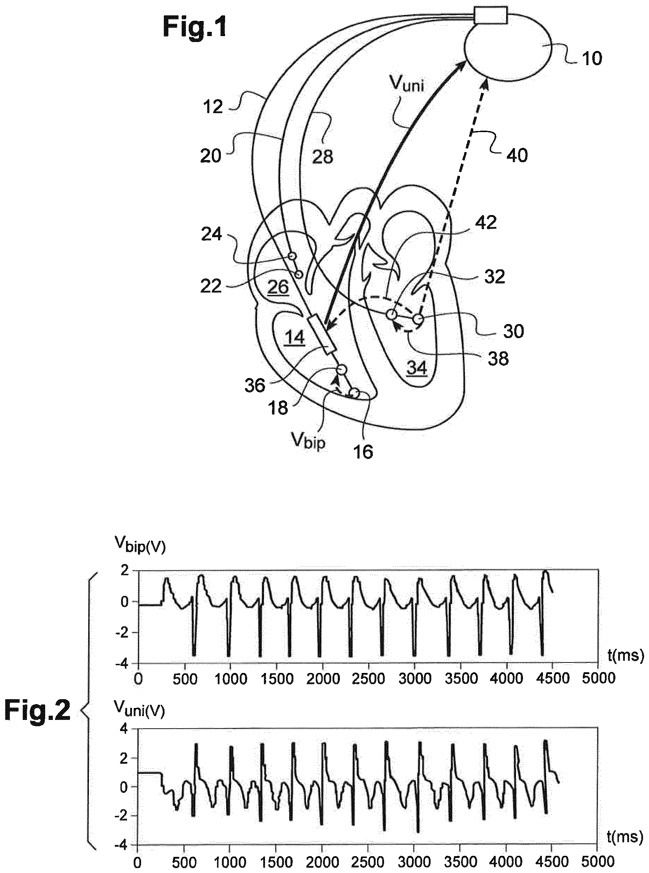

La Figure 1 illustre une configuration de stimulation dans laquelle un générateur d'impulsions 10 est associé à une première sonde 12 implantée dans le ventricule droit 14. La tête de cette sonde comporte deux électrodes, à savoir une électrode distale (*tip*) 16 et une électrode proximale (*ring*) 18. Une deuxième sonde 20 est pourvue d'électrodes auriculaires de détection distale 22 et proximale 24 situées au niveau de l'oreillette droite 26 pour la détection des signaux dans cette cavité et l'application éventuelle d'une stimulation auriculaire.

Pour permettre la stimulation biventriculaire, notamment afin de rétablir la synchronisation entre les deux ventricules, le dispositif est pourvu d'une troisième sonde 28, par exemple une sonde disposée dans le réseau coronaire, comportant une ou plusieurs électrodes 30, 32 disposées au voisinage du ventricule gauche 34. Outre les électrodes distale et proximale 30, 32 illustrées, la sonde gauche peut également comporter une ou plusieurs électrodes intermédiaires située(s) dans une position médiane entre les électrodes 30 et 32.

Il est ainsi possible d'assurer la stimulation concomitante, ou avec un léger décalage temporel contrôlé (délai interventriculaire DW), des deux ventricules droit et gauche pour rétablir la synchronisation entre ces deux cavités et améliorer l'hémodynamique générale du patient. La sonde ventriculaire droite 12 peut également être pourvue d'un bobinage (*coil*) ventriculaire 36 formant électrode de défibrillation et permettant aussi de recueillir un signal endocavitaire (ce bobinage pouvant également remplacer l'électrode proximale *ring* 18).

En ce qui concerne spécifiquement la stimulation du ventricule gauche, il est possible d'utiliser une configuration bipolaire (entre les deux électrodes 30 et 32 de la sonde 28) ou unipolaire (entre l'une des électrodes 30 ou 32 et le boitier *can*) du générateur 10. Les deux "vecteurs de stimulation" correspondants sont référencés 38 et 40 sur la Figure 1. Ces mêmes vecteurs peuvent être utilisés également pour le recueil d'un signal de dépolarisation ventriculaire gauche. On notera que dans le cas d'une sonde multipolaire il existe un grand nombre de vecteurs bipolaires et unipolaires possibles, définis à partir de chacune des électrodes.

Le but de l'invention est de réaliser un monitorage du remodelage cardiaque, à partir de la mesure du temps de conduction interventriculaire :
- lorsque le volume ventriculaire diminue (remodelage inverse, bénéfique pour le patient), le délai de conduction entre les ventricules devrait se raccourcir ;
- inversement, lorsque le coeur se dilate (remodelage, délétère), le temps de conduction entre les ventricules devrait s'allonger.

Plus précisément, pour pallier les inconvénients des techniques connues qui ont été rappelées en introduction, l'invention propose de combiner deux signaux d'électrogramme endocavitaire (EGM) issus de la même cavité ventriculaire, par exemple issus du ventricule droit, ces signaux résultant de la dépolarisation produite suite à l'application d'une impulsion de stimulation sur l'autre ventricule - le ventricule gauche dans cet exemple.

Comme illustré Figure 1, les EGMs ainsi recueillis dans le ventricule droit peuvent comprendre :
- une composante ventriculaire droite *Vbip,* dérivée d'un signal EGM *near-field* bipolaire recueilli entre l'électrode distale 16 et l'électrode proximale 18 de la sonde ventriculaire droite 12, et
- une autre composante ventriculaire droite *Vuni,* dérivée d'un signal EGM *far-field* unipolaire recueilli entre le bobinage de défibrillation 36 de la sonde ventriculaire droite 12 et le boitier métallique du générateur 10.

La Figure 2 illustre un exemple de tracés d'EGMs *Vbip* et *Vuni* observés respectivement sur la voie bipolaire ventriculaire et la voie unipolaire ventriculaire de la configuration de la Figure 1.

D'autres configurations peuvent être utilisées, à partir de signaux de type *far-field* (par exemple entre l'une des électrodes 16 ou 18 et le boitier 10) et de type *near-field* provenant de la cavité ventriculaire non stimulée, à savoir le ventricule droit dans l'exemple illustré.

On pourrait également envisager, dans une configuration inverse, de stimuler le ventricule droit et recueillir les EGMs bipolaire et unipolaire dans le ventricule gauche. La composante bipolaire peut alors être recueillie par exemple entre les électrodes 30 et 32 de la sonde ventriculaire gauche 28 (comme référencé en 38), et la composante unipolaire entre l'électrode *tip* 30 (ou l'électrode *ring* 32) et le boitier can du générateur 10, comme référencé en 40. En variante, la composante unipolaire peut être également recueillie entre l'électrode *tip* 30 (ou l'électrode *ring* 32) de la sonde ventriculaire gauche 28 et le bobinage *coil* 36 de la sonde ventriculaire droite 12, comme référencé en 42.

Les Figures 3 et 4 correspondent à deux exemples de signaux EGM *Vbip* et *Vuni* recueillis avec la configuration de la Figure 1, tels qu'ils se présentent au moment de l'implantation (courbe M0), trois mois après l'implantation (courbe M3) et un an après l'implantation (courbe M12).

Comme on peut le voir, la forme de ces signaux évolue au cours du temps, mais par exemple dans le cas de la Figure 3 le signal unipolaire n'apporte pas vraiment d'information sur l'évolution dans le temps du délai de conduction, alors que le signal bipolaire *Vbip* contient des informations beaucoup plus significatives. Inversement, dans le cas de la Figure 4, la réponse est très tardive sur le signal bipolaire *Vbip* et c'est la voie unipolaire (entre le bobinage *coil* 36 et le boitier *can* 10 du générateur) qui donne l'information recherchée, de façon beaucoup plus discriminante. Pour pallier cet inconvénient, l'invention propose de combiner ces deux composantes bipolaire et unipolaire en une caractéristique unique contenant, de façon plus globale, toute l'information disponible afin de pouvoir procéder à une évaluation complète et robuste de l'état du patient au fil du temps.

Cette combinaison des deux signaux bipolaire et unipolaire est réalisée sous forme d'une "boucle cardiaque" ou "vectogramme" (VGM), qui est la représentation dans un espace à deux dimensions de l'un des deux signaux EGM (en ordonnée) par rapport à l'autre (en abscisse). Chaque cycle cardiaque est alors représenté par un vectogramme dans le plan {*Vbip,Vuni*} ainsi défini, vectogramme qui fait donc abstraction de la dimension temporelle.

On soulignera que ce "vectogramme" (VGM), qui est obtenu à partir de signaux d'électrogramme (EGM) issu de sondes intracardiaques, ne doit pas être confondu avec le "vectocardiogramme" (VCG) qui est, lui, obtenu à partir de signaux d'électrocardiogramme (ECG) issu d'électrodes externes placées sur le thorax du patient.

La construction d'un VGM et son analyse pour quantifier des données cardiaques sont décrites par exemple dans Milpied et al., "Arrhythmia Discrimination in Implantable Cardioverter Defibrillators using Support Vector Machines applied to a new Représentation of Electrograms," IEEE Transactions on Biomedical Engineering, June 2011, 58(6):1797-1803. L'analyse d'un VGM à des fins différentes de celles de l'invention a été déjà proposée notamment par le EP 2 105 843 (ELA Medical) pour la discrimination entre tachycardies ventriculaires et supraventriculaires, par le EP 2 324 885 A1 (Sorin CRM) pour l'invalidation d'un test de capture en cas de fusion, c'est-à-dire de stimulation déclenchée de façon concomitante à une dépolarisation spontanée, par le EP 2 368 493 A1 (Sorin CRM) pour la discrimination d'artefacts de bruit pour la validation ou l'invalidation de cycles cardiaques à analyser, par le EP 2 742 971 A1 (Sorin CRM) pour déterminer la présence ou l'absence d'une onde évoquée induite par la stimulation d'une cavité, ou encore par le EP 2 742 973 A1

(Sorin CRM) pour détecter la présence éventuelle d'un phénomène de stimulation anodique.

Concrètement, comme illustré Figure 5, les signaux EGM *Vuni*(*t*) et *Vbip*(*t*) recueillis sont échantillonnés, et les échantillons successifs des deux composantes sont mémorisés puis combinés entre eux pour produire une courbe paramétrique (la caractéristique VGM) du type VGM = (V*bip*(t),V*uni*(t)) ou {x = V*bip*(t), y = V*uni*(t)}.

Cette courbe est une courbe paramétrée par le temps, tracée à partir des variations de l'une des composantes temporelles (*Vuni*) en fonction de l'autre (*Vbip*). Elle constitue un vectogramme (VGM) représentatif du cycle cardiaque à analyser, et sera également désignée "caractéristique 2D paramétrique". Elle présente graphiquement la forme d'une boucle, le temps n'apparaissant plus que dans la manière dont la boucle est parcourue sur la durée du cycle.

On notera incidemment que l'analyse 'bidimensionnelle" ou "en deux dimensions" (2D) évoquée ici ne doit pas être entendue de manière en elle-même limitative. L'invention peut en effet s'appliquer aussi bien à une analyse dans un espace multidimensionnel d'ordre supérieur (3D ou plus), par extrapolation des enseignements de la présente description à une situation où des signaux EGM provenant d'une même cavité sont recueillis simultanément sur trois voies ou plus.

En pratique, comme illustré Figure 6, l'échantillonnage produit un VGM en forme de polygone ouvert où chaque sommet correspond à un point d'échantillonnage de la mesure du signal *Vuni* et *Vbip* de l'EGM. Dans l'exemple de la Figure 6, l'échantillonnage est opéré à une fréquence de 128 Hz, ce qui donne environ 20 à 25 points de mesure, qui sont autant de valeurs qui peuvent être mises en mémoire pour être analysées.

La Figure 7 illustre plus précisément la forme des composantes *Vbip* et *Vuni* dans le cas d'une stimulation du seul ventricule gauche, le VGM étant construit avec des EGMs *Vbip* et *Vuni* recueillis dans le ventricule droit. Sur cette figure on peut voir notamment les pics de signal *stimA* correspondant à la stimulation auriculaire et *stimVG* correspondant à la stimulation ventriculaire, ainsi que le temps de conduction interventriculaire ΔVV.

Pour l'analyse, le VGM est enregistré à partir de l'instant du pic de stimulation ventriculaire *stimVG.* Avantageusement, le délai atrioventriculaire DAV séparant la stimulation auriculaire de la stimulation ventriculaire est réduit pendant le temps de la mesure à une valeur minimale, par exemple DAV = 30 ms, pour s'assurer qu'il n'y aura pas de phénomène de fusion avec une conduction spontanée dans le ventricule où a lieu la détection (le ventricule droit). L'oreillette peut également être stimulée pendant le temps de la mesure pour allonger artificiellement le délai PR et éviter une fusion.

Le VGM est construit périodiquement, par exemple une fois par jour ou une fois par semaine, dans des conditions stables (rythme sinusal lent, de préférence la nuit), pour être ensuite mémorisé et comparé à un VGM de référence créé à l'initialisation de la thérapie, et éventuellement mis à jour en cas d'alerte ou de décompensation, ou de façon manuelle par le praticien lors d'une visite de suivi par exemple.

Avantageusement, la caractéristique VGM est mémorisée sous forme d'un vecteur de paramètres descripteurs intrinsèques, décrivant sous forme condensée les caractéristiques importantes qui serviront à l'analyse de la courbe VGM.

Avantageusement, ces paramètres descripteurs intrinsèques du VGM sont, comme illustré Figure 8, les valeurs successives de la norme de la vitesse de parcours du VGM, calculée en chaque point de ce VGM. En un point donné, la vitesse est une donnée vectorielle (la vitesse étant définie par son orientation et sa norme) qui, pour une caractéristique échantillonnée, peut être calculée à partir du point précédent et du point suivant sur la courbe.

En pratique, pour le but recherché, à savoir le suivi de l'évolution du temps de conduction interventriculaire, il apparait que la norme de la vitesse est un paramètre suffisant, ce qui permettra une économie importante en terme de moyens de calcul et de mémoire, un VGM donné étant simplement mémorisé sous forme d'une série (vecteur) de paramètres descripteurs intrinsèques représentatifs du VGM courant construit à une date donnée.

Pour comparer les VGMs courants (mémorisés sous forme d'un vecteur de descripteurs intrinsèques) avec un VGM de référence (mémorisé sous forme d'un vecteur homologue de paramètres descripteurs), on opère avantageusement une corrélation croisée entre ces deux vecteurs de descripteurs.

La Figure 9 représente schématiquement une telle corrélation croisée : la fonction schématisée *f* correspond au vecteur des paramètres descripteurs intrinsèques du VGM de référence et la fonction *g* au vecteur des paramètres descripteurs intrinsèques du VGM courant à comparer. La corrélation croisée *f***g* produit une valeur τ d'un décalage à appliquer à l'un des vecteurs de paramètres descripteurs afin de s'aligner sur l'autre vecteur de paramètres descripteurs.

La valeur du délai τ qui maximise la fonction d'intercorrélation *f***g*(τ) est considérée comme étant un indice représentatif de l'état du patient à la date de construction du VGM courant considéré :
- si ce délai τ est négatif, cela signifie qu'un retard doit être appliqué au VGM courant pour maximiser la fonction d'intercorrélation, le temps de conduction s'étant raccourci. Si cette valeur dépasse un seuil prédéterminé, par exemple τ < -10 ms, on considère alors que l'on est en présence d'un remodelage inverse, bénéfique ;
- inversement, si ce délai τ est positif, cela signifie que le retard doit être appliqué au VGM de référence et que le temps de conduction s'est allongé, signifiant une aggravation de l'état du patient. De la même façon, si cette valeur dépasse un seuil prédéterminé, par exemple τ > 10 ms, on considère qu'il y a remodelage.

La Figure 10 illustre de façon très générale la manière dont est mis en oeuvre le dispositif de l'invention.

À partir des EGMs bipolaire et unipolaire recueillis, un VGM est construit (bloc 100). Dans un premier temps, une référence est constituée (bloc 102, détaillé Figure 11) et conservée en mémoire (bloc 104). De la même façon, périodiquement, par exemple quotidiennement, un VGM courant est construit et comparé à la référence mémorisée (bloc 106) pour en déduire un indicateur de l'évolution de l'état du patient : remodelage, remodelage inverse, ou état non évolutif.

La création de la référence (bloc 102 de la Figure 10) est opérée comme illustré Figure 11.

Cette référence est créée à partir de plusieurs cycles cardiaques représentatifs (absence d'artefact, d'extrasystole, de fusion, etc.), par exemple x = 2 à 5 cycles cardiaques, pour chacun desquels le VGM est construit et le vecteur de paramètres descripteurs est calculé (bloc 108). Ces données sont ensuite moyennées (bloc 110) ou, en variante, l'un des cycles cardiaques le plus représentatif est sélectionné pour constituer la référence (bloc 112). À partir du VGM moyenné ou sélectionné, la référence est validée (bloc 114), en la comparant à chacun des cycles recueillis à l'étape 108, de manière à vérifier que l'écart entre cette référence et chacun des autres cycles reste dans des limites acceptables.

La Figure 12 illustre la séquence des différentes opérations du test du VGM courant (bloc 106 de la Figure 10) effectué à intervalles réguliers, par exemple chaque jour.

Le patient est stimulé pendant quelques cycles cardiaques non plus en stimulation CRT biventriculaire mais en stimulation du seul ventricule gauche, avec un DAV réduit au minimum pour éviter tout risque de fusion (par exemple DAV = 30 ms) et une détection de l'onde évoquée dans le ventricule droit, à la fois selon une composante bipolaire *Vbip* et une composante unipolaire *Vuni* (bloc 118).

Si aucune référence n'est disponible à ce stade (c'est-à-dire juste après l'implantation), ou si les paramètres de stimulation ont été modifiés, il convient de créer une référence correspondant à l'état actuel du patient. Pour ce faire, un VGM de référence est construit (de la manière expliquée plus haut en référence à la Figure 11), par exemple, dans le cas d'un échantillonnage à 128 Hz, sur une durée d'environ 125 ms suivant la stimulation auriculaire, à partir des échantillons n° 10 à 26 (bloc 120). Les valeurs de la norme de la vitesse en chaque point du VGM sont ensuite calculées et mémorisées pour construire un vecteur de paramètres descripteurs intrinsèques courants du VGM, qui est dans cet exemple constitué d'un vecteur de 17 valeurs (bloc 122).

Si à l'issue de l'étape 118 une référence est disponible en mémoire, alors le dispositif construit le VGM courant (bloc 124) et constitue le vecteur de paramètres descripteurs intrinsèques courants correspondants (bloc 126) de la même manière qu'aux blocs 120 et 122 décrits plus haut.

Une corrélation croisée est ensuite opérée entre les paramètres descripteurs courants issus du bloc 126 et les paramètres descripteurs de référence qui avaient été calculés auparavant au bloc 122, afin d'obtenir la valeur τ de retard qui maximise la fonction de corrélation croisée entre le vecteur de paramètres descripteurs courants et le vecteur de paramètres descripteurs de référence.

Si cette valeur de retard τ est positive et supérieure à un seuil donné, par exemple τ > 10 ms, ceci indique qu'il y a eu décompensation et remodelage (bloc 130), révélant une aggravation de l'état du patient. Une alerte peut être déclenchée afin que des mesures appropriées puissent être prises sans délai : modification des paramètres de stimulation CRT, prise d'un traitement médicamenteux, etc.

Si le retard τ est relativement faible, par exemple -10 ms < τ < 10 ms, on considère que l'état du patient n'a pas évolué de façon significative (bloc 132).

Enfin, pour des valeurs de τ négatives en deçà d'un seuil donné, par exemple τ < -10 ms, on considère qu'il y a eu remodelage inverse (bloc 134) et donc amélioration de l'état du patient.

## Revendications

1. Un dispositif médical implantable actif (10) de resynchronisation cardiaque par stimulation biventriculaire, comportant :
- des moyens de détection d'événements auriculaires et ventriculaires ;
- des moyens de stimulation des ventricules droit et gauche, avec application i) d'un délai atrioventriculaire, DAV, entre un événement auriculaire spontané ou stimulé et une stimulation du ventricule droit, et ii) d'un délai interventriculaire, DW, entre les instants respectifs de stimulation des ventricules droit et gauche ;
- des moyens de mesure multidimensionnelle du temps de conduction interventriculaire, compté entre une stimulation du ventricule droit (14) et un événement spontané détecté dans le ventricule gauche (34), ouvice versa, ou entre une stimulation du ventricule droit (14) ou gauche (34) et un événement spontané détecté entre ventricules droit et gauche, au cours d'un même cycle cardiaque ; et
- des moyens de suivi de l'évolution d'un état du patient, configurés pour mémoriser un historique des temps de conduction mesurés à des dates successives et à en dériver un indice représentatif, où les moyens de mesure multidimensionnelle du temps de conduction interventriculaire comprennent :
- des moyens configurés pour commander pendant au moins un cycle cardiaque les moyens de stimulation et les moyens de détection de manière à :
• produire une stimulation de l'un des ventricules, et
• recueillir dans l'autre ventricule, concurremment sur des voies respectives distinctes, au moins deux signaux EGM d'électrogramme endocavitaire et en dériver au moins deux composantes temporelles distinctes (Vbip, Vuni) respectives ;
- des moyens configurés pour combiner les au moins deux composantes temporelles en au moins une caractéristique 2D paramétrique (VGM) représentative du cycle cardiaque, à partir des variations de l'une des composantes temporelles en fonction de l'autre ;
- et **caractérise en ce que** les moyens de mesure multidimensionelle du temps de conduction intraventriculaire comprennent aussi : des moyens d'analyse, configurés pour dériver de la caractéristique 2D, ou d'une moyenne de caractéristiques 2D recueillies sur des cycles cardiaques successifs, un vecteur de paramètres descripteurs intrinsèques courants de la caractéristique 2D ; et
- des moyens comparateurs, configurés pour comparer ledit vecteur de paramètres descripteurs courants à un vecteur homologue de paramètres descripteurs de référence, pour en dériver un indice représentatif de l'évolution d'un état du patient.

2. Le dispositif de la revendication 1, dans lequel lesdits paramètres descripteurs intrinsèques sont des paramètres fonction du vecteur vitesse de la caractéristique 2D, considéré en une pluralité de points respectifs de cette caractéristique.

3. Le dispositif de la revendication 2, dans lequel lesdits paramètres fonction du vecteur vitesse sont les valeurs respectives de la norme du vecteur vitesse.

4. Le dispositif de la revendication 1, dans lequel les moyens comparateurs comprennent des moyens configurés pour opérer une corrélation croisée (f* g) entre i) le vecteur de paramètres descripteurs courants (f) et ii) le vecteur de paramètres descripteurs de référence (g).

5. Le dispositif de la revendication 4, dans lequel ledit indice représentatif de l'évolution d'un état du patient est la valeur du retard (τ) qui maximise la fonction d'intercorrélation entre le vecteur de paramètres descripteurs courants et le vecteur de paramètres descripteurs de référence.

6. Le dispositif de la revendication 5, comprenant en outre des moyens configurés pour comparer ledit retard à un seuil prédéterminé et à déclencher une alerte en cas de franchissement de ce seuil.

7. Le dispositif de la revendication 1, dans lequel le dispositif comprend en outre des moyens configurés pour raccourcir temporairement le délai atrioventriculaire du dispositif pendant l'activation des moyens de mesure multidimensionnelle du temps de conduction interventriculaire.

8. Le dispositif de la revendication 1, dans lequel le dispositif comprend en outre des moyens configurés pour forcer temporairement l'activation de moyens de stimulation auriculaires pendant l'activation des moyens de mesure multidimensionnelle du temps de conduction interventriculaire.

9. Le dispositif de la revendication 1, dans lequel lesdits au moins deux signaux EGM recueillis concurremment sur des voies respectives distinctes comprennent :
- un signal EGMfar-field unipolaire (Vuni) recueilli entre i) une électrode proximale (18) ou distale (16) ou le cas échéant une électrode intermédiaire ou un bobinage de défibrillation (36) d'une sonde ventriculaire située dans le ventricule non stimulé et ii) un boitier métallique de générateur (10) du dispositif,
ou bien entre i) une première électrode proximale, ou électrode distale ou bobinage de défibrillation et ii) une seconde électrode proximale, ou une électrode distale ou le cas échéant une électrode intermédiaire ou un bobinage de défibrillation, respectivement de deux sondes ventriculaires distinctes situées toutes deux dans le ventricule non stimulé, ou bien entre i) une première électrode proximale, ou une électrode distale ou un bobinage de défibrillation et ii) une seconde électrode proximale, ou une électrode distale ou une électrode intermédiaire, respectivement d'une sonde ventriculaire droite et d'une sonde ventriculaire gauche, et
- un signal EGMnear-field bipolaire (Vbip) recueilli entre i) une électrode distale (16) et ii) une électrode proximale (18) d'une sonde ventriculaire située dans le ventricule non stimulé,
ou bien entre i) un bobinage de défibrillation (36) et ii) une électrode distale (16) ou proximale (18) de ladite sonde ventriculaire (12),
ou bien entre i) une électrode distale et ii) une électrode intermédiaire d'une sonde ventriculaire gauche (28),
ou bien entre i) une électrode proximale et ii) une électrode intermédiaire de ladite sonde ventriculaire gauche (28),
ou bien entre deux électrodes intermédiaires de ladite sonde ventriculaire gauche (28).

## Patentansprüche

1. Aktive implantierbare medizinische Vorrichtung (10) für die Herzsynchronisation durch biventrikuläre Stimulation, die Folgendes umfasst:
- Mittel zum Detektieren von aurikulären und ventrikulären Ereignissen;
- Mittel zum Stimulieren des rechten und des linken Ventrikels durch Anwenden i) einer atrioventrikulären Verzögerung DAV zwischen einem spontanen oder stimulierten aurikulären Ereignis und einer Stimulation des rechten Ventrikels und ii) einer interventrikulären Verzögerung DVV zwischen den jeweiligen Zeitpunkten der Stimulation des rechten bzw. des linken Ventrikels;
- Mittel zum mehrdimensionalen Messen der interventrikulären Leitungszeit, die zwischen einer Stimulation des rechten Ventrikels (14) und einem in dem linken Ventrikel (34) detektierten spontanen Ereignis gezählt wird, oder umgekehrt, oder zwischen einer Stimulation des rechten Ventrikels (14) oder des linken Ventrikels (34) und einem zwischen dem rechten und dem linken Ventrikel detektierten spontanen Ereignis während desselben Herzzyklus gezählt wird; und
- Mittel zum Verfolgen der Entwicklung eines Zustands des Patienten, die konfiguriert sind, eine Historie der Leitungszeiten, die an aufeinander folgenden Daten gemessen werden, zu speichern und daraus eine repräsentative Angabe abzuleiten, wobei die Mittel zum mehrdimensionalen Messen der interventrikulären Leitungszeit Folgendes umfassen:
- Mittel, die konfiguriert sind, wenigstens während eines Herzzyklus die Stimulationsmittel und die Detektionsmittel in der Weise zu steuern, dass:
• eine Stimulation eines der Ventrikel erzeugt wird und
• in dem anderen Ventrikel gleichzeitig auf jeweils verschiedenen Wegen wenigstens zwei EGM-Signale eines endokavitären Elektrogramms empfangen werden und daraus wenigstens zwei jeweilige verschiedene Zeitkomponenten (Vbip, Vuni) abgeleitet werden;
- Mittel, die konfiguriert sind, die wenigstens zwei zeitlichen Komponenten zu wenigstens einer parametrischen 2D-Charakteristik (VGM), die den Herzzyklus repräsentiert, ausgehend von Veränderung einer der zeitlichen Komponenten als Funktion der anderen zu kombinieren;
- und **dadurch gekennzeichnet, dass** die Mittel zum mehrdimensionalen Messen der intraventrikulären Leitungszeit außerdem Folgendes umfassen:
- Analysemittel, die konfiguriert sind, aus der 2D-Charakteristik oder aus einem Mittelwert von 2D-Charakteristiken, die in aufeinander folgenden Herzzyklen empfangen werden, einen Vektor aktueller intrinsischer Deskriptorparameter der 2D-Charakteristik abzuleiten; und
- Komparatormittel, die konfiguriert sind, den Vektor aktueller Deskriptorparameter mit einem homologen Vektor von Referenz-Deskriptorparametern zu vergleichen, um daraus eine Angabe abzuleiten, die die Entwicklung eines Zustands des Patienten repräsentiert.

2. Vorrichtung nach Anspruch 1, wobei die intrinsischen Deskriptorparameter Parameter sind, die eine Funktion des Geschwindigkeitsvektors der 2D-Charakteristik sind, die an mehreren jeweiligen Punkten dieser Charakteristik betrachtet werden.

3. Vorrichtung nach Anspruch 2, wobei die Parameter, die eine Funktion des Geschwindigkeitsvektors sind, jeweilige Werte der Norm des Geschwindigkeitsvektors sind.

4. Vorrichtung nach Anspruch 1, wobei die Komparatormittel Mittel umfassen, die konfiguriert sind, eine Kreuzkorrelation (f*g) zwischen i) dem aktuellen Deskriptorparameter-Vektor (f) und ii) dem Vektor von Referenz-Deskriptorparametern (g) auszuführen.

5. Vorrichtung nach Anspruch 4, wobei die Angabe, die die Entwicklung eines Zustands des Patienten repräsentiert, der Wert einer Verzögerung (τ) ist, der die Interkorrelationsfunktion zwischen dem Vektor aktueller Deskriptorparameter und dem Vektor von Referenz-Deskriptorparametern maximal macht.

6. Vorrichtung nach Anspruch 5, die außerdem Mittel umfasst, die konfiguriert sind, die Verzögerung mit einem vorgegebenen Schwellenwert zu vergleichen und im Fall eines Überschreitens dieses Schwellenwerts eine Warnung auszugeben.

7. Vorrichtung nach Anspruch 1, wobei die Vorrichtung außerdem Mittel umfasst, die konfiguriert sind, die atrioventrikuläre Verzögerung der Vorrichtung während der Aktivierung der Mittel zum mehrdimensionalen Messen der interventrikulären Leitungszeit vorübergehend zu verkürzen.

8. Vorrichtung nach Anspruch 1, wobei die Vorrichtung außerdem Mittel umfasst, die konfiguriert sind, die Aktivierung von aurikulären Stimulationsmitteln während der Aktivierung der Mittel zum mehrdimensionalen Messen der interventrikulären Leitungszeit vorübergehend zu erzwingen.

9. Vorrichtung nach Anspruch 1, wobei die wenigstens zwei EGM-Signale, die gleichzeitig auf jeweils verschiedenen Wegen empfangen werden, Folgendes umfassen:
- ein unipolares EGM-Fernfeldsignal (Vuni), das zwischen i) einer proximalen Elektrode (18) oder einer distalen Elektrode (16) oder gegebenenfalls einer Zwischenelektrode oder einer Defibrillationswicklung (36) einer ventrikulären Sonde, die sich in dem nicht stimulierten Ventrikel befindet, und ii) einem Generatormetallgehäuse (10) der Vorrichtung,
oder zwischen i) einer ersten proximalen Elektrode oder distalen Elektrode oder Defibrillationswicklung und ii) einer zweiten proximalen Elektrode oder distalen Elektrode oder gegebenenfalls einer Zwischenelektrode oder einer Defibrillationswicklung von zwei entsprechenden, verschiedenen ventrikulären Sonden, die sich beide in dem nicht stimulierten Ventrikel befinden,
oder zwischen i) einer ersten proximalen Elektrode oder einer distalen Elektrode oder einer Defibrillationswicklung und ii) einer zweiten proximalen Elektrode oder einer distalen Elektrode oder einer Zwischenelektrode einer rechten ventrikulären Sonde bzw. einer linken ventrikulären Sonde
empfangen wird, und
- ein bipolares EGM-Nahfeldsignal (Vbip), das zwischen i) einer distalen Elektrode (16) und ii) einer proximalen Elektrode (18) einer ventrikulären Sonde, die sich in dem nicht stimulierten Ventrikel befindet,
oder zwischen i) einer Defibrillationswicklung (36) und ii) einer distalen Elektrode (16) oder proximalen Elektrode (18) der ventrikulären Sonde (12)
oder zwischen i) einer distalen Elektrode und ii) einer Zwischenelektrode einer linken ventrikulären Sonde (28),
oder zwischen i) einer proximalen Elektrode und ii) einer Zwischenelektrode der linken ventrikulären Sonde (28)
oder zwischen zwei Zwischenelektroden der linken ventrikulären Sonde (28) empfangen wird.

## Claims

1. Active implantable medical device (10) for heart resynchronization through biventricular stimulation, including:
- means for detecting atrial and ventricular events;
- means for stimulating the right and left ventricles, with the application i) of an atrioventricular delay, DAV, between a spontaneous or stimulated atrial event and a stimulation of the right ventricle, and ii) of an interventricular delay, DVV, between the respective times at which the right and left ventricles are stimulated;
- means for multidimensionally measuring the interventricular conduction time, counted between a stimulation of the right ventricle (14) and a spontaneous event detected in the left ventricle (34), or vice versa, or between a stimulation of the right (14) or left (34) ventricle and a spontaneous event detected between right and left ventricles, during one and the same cardiac cycle; and
- means for tracking the variation in a state of the patient, which means are configured to store a history of the conduction times measured at successive dates and to derive therefrom a representative index,
the means for multidimensionally measuring the interventricular conduction time comprising:
- means that are configured to control, for at least one cardiac cycle, the stimulation means and the detection means so as:
• to bring about a stimulation of one of the ventricles; and
• to receive, concurrently over distinct respective channels, at least two intracardiac electrogram EGM signals from the other ventricle and to derive therefrom at least two respective distinct temporal components (Vbip, Vuni);
- means that are configured to combine the at least two temporal components into at least one parametric 2D characteristic (VGM) that is representative of the cardiac cycle on the basis of the variations in one of the temporal components with respect to the other,
and **characterized in that** the means for multidimensionally measuring the interventricular conduction time also comprise:
- analysis means, configured to derive the 2D characteristic, or a mean of 2D characteristics that are received over successive cardiac cycles, a vector of current intrinsic descriptive parameters of the 2D characteristic; and
- comparing means, configured to compare said vector of current descriptive parameters with a homologous vector of reference descriptive parameters so as to derive therefrom an index that is representative of the variation in a state of the patient.

2. The device of Claim 1, wherein said intrinsic descriptive parameters are parameters according to the velocity vector of the 2D characteristic, considered at a plurality of respective points of this characteristic.

3. The device of Claim 2, wherein said parameters according to the velocity vector are the respective values of the norm of the velocity vector.

4. The device of Claim 1, wherein the comparing means comprise means that are configured to carry out a cross-correlation (f* g) between i) the vector of current descriptive parameters (f) and ii) the vector of reference descriptive parameters (g).

5. The device of Claim 4, wherein said index that is representative of the variation in a state of the patient is the value of the delay (τ) that maximizes the cross-correlation function between the vector of current descriptive parameters and the vector of reference descriptive parameters.

6. The device of Claim 5, further comprising means that are configured to compare said delay with a predetermined threshold and to issue an alert in the event that this threshold is crossed.

7. The device of Claim 1, wherein the device further comprises means that are configured to temporarily shorten the atrioventricular delay of the device during the activation of the means for multidimensionally measuring the interventricular conduction time.

8. The device of Claim 1, wherein the device further comprises means that are configured to temporarily force the activation of the atrial stimulation means during the activation of the means for multidimensionally measuring the interventricular conduction time.

9. The device of Claim 1, wherein said at least two EGM signals that are received concurrently over distinct respective channels comprise:
- a unipolar far-field EGM signal (Vuni) received between i) a proximal (18) or distal (16) electrode or, if applicable, an intermediate electrode or a defibrillator coil (36) of a ventricular probe that is located in the unstimulated ventricle and ii) a metal generator housing (10) of the device,
or else between i) a first proximal electrode, distal electrode or defibrillator coil and ii) a second proximal electrode, a distal electrode or, if applicable, an intermediate electrode or defibrillator coil, respectively of two distinct ventricular probes that are both located in the unstimulated ventricle,
or else between i) a first proximal electrode, a distal electrode or a defibrillator coil and ii) a second proximal electrode, a distal electrode or an intermediate electrode, respectively of a right ventricular probe and of a left ventricular probe; and
- a bipolar near-field EGM signal (Vbip) received between i) a distal electrode (16) and ii) a proximal electrode (18) of a ventricular probe that is located in the unstimulated ventricle,
or else between i) a defibrillator coil (36) and ii) a distal (16) or proximal (18) electrode of said ventricular probe (12),
or else between i) a distal electrode and ii) an intermediate electrode of a left ventricular probe (28),
or else between i) a proximal electrode and ii) an intermediate electrode of said left ventricular probe (28),
or else between two intermediate electrodes of said left ventricular probe (28).
